(19) Europäisches Patentamt
European Patent Office
Office européen des brevets

(11) Veröffentlichungsnummer: **0 069 881**
**A1**

# (12) EUROPÄISCHE PATENTANMELDUNG

(21) Anmeldenummer: 82105413.7

(22) Anmeldetag: 21.06.82

(51) Int. Cl.³: C 07 F 9/65
A 01 N 57/16

(30) Priorität: 03.07.81 DE 3126390

(43) Veröffentlichungstag der Anmeldung: 19.01.83 Patentblatt 83/3

(84) Benannte Vertragsstaaten: AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Maurer, Fritz, Dr.
Roeberstrasse 8
D-5600 Wuppertal 1(DE)

(72) Erfinder: Hammann, Ingeborg, Dr.
Belfortstrasse 9
D-5000 Köln 1(DE)

(72) Erfinder: Stendel, Wilhelm, Dr.
In den Birken 55
D-5600 Wuppertal 1(DE)

## (54) S-Azolyl-methyl-di(tri)-thiophosphorsäureester, Verfahren zu ihrer Herstellung und ihre Verwendung als Schädlingsbekämpfungsmittel.

(57) Die Erfindung betrifft neue S-Azolyl-methyl-di(tri)-thiophosphorsäureester (Formel I) und ihre Salze, Verfahren zu ihrer Herstellung, sie enthaltende Schädlingsbekämpfungsmittel, ihre Herstellung und Verwendung als Schädlingsbekämpfungsmittel, insbesondere als Insektizide und Akarizide.

Die neuen Verbindungen der Formel I

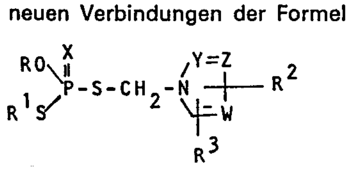

(I)

in welcher

R und $R^1$ gleich oder verschieden sind und für gegebenenfalls substituiertes Alkyl stehen,

$R^2$ und $R^3$ gleich oder verschieden sind und für Wasserstoff, Halogen oder gegebenenfalls substituierte Alkyl-, Alkoxy-, Alkylthio- oder Arylreste stehen,

Y, Z und W gleich oder verschieden sind und für Stickstoff oder einen gegebenenfalls durch $R^2$ oder $R^3$ substituierten Methinrest stehen, und

X für Sauerstoff oder Schwefel steht,

und ihre Salze werden z.B. dadurch erhalten, daß man Azolyl-methyl-halogenide der Formel II

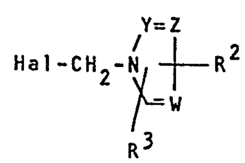

(II)

in welcher

$R^2$, $R^3$, W, Y und Z die oben angegebene Bedeutung haben, und

Hal für Halogen steht,

mit Phosphorsäureester-Salzen der Formel III

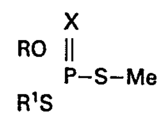

(III)

in welcher

R, $R^1$ und X die oben angegebenen Bedeutungen haben, und

Me für Alkali- oder Erdalkaliionen oder Ammonium steht,

gegebenenfalls in Gegenwart von Verdünnungsmitteln umsetzt und man gewünschtenfalls die Salze herstellt.

EP 0 069 881 A1

0069881

BAYER AKTIENGESELLSCHAFT 5090 Leverkusen, Bayerwerk

Zentralbereich S/Fr
Patente, Marken und Lizenzen I b 02. Juli 1981

# S-Azolyl-methyl-di(tri)-thiophosphorsäureester, Verfahren zu ihrer Herstellung und ihre Verwendung als Schädlingsbekämfungsmittel

Die Erfindung betrifft neue S-Azolyl-methyl-di(tri)-thiophosphorsäureester und ihre Salze, Verfahren zu ihrer Herstellung, sie enthaltende Schädlingsbekämpfungsmittel, ihre Herstellung und Verwendung als Schädlingsbekämpfungsmittel, insbesondere als Insektizide und Akarizide.

Es ist bekannt, daß bestimmte O,O-Dialkyl-S-azolyl-methyl-dithiophosphorsäureester, wie z.B. O,O-Diethyl-S-(3,5-dimethyl-pyrazol-1-yl)-methyl- und O,O-Dimethyl-S-(3,5-dimethyl-pyrazol-1-yl)-methyl-dithiophosphorsäureester insektizide Eigenschaften aufweisen (vergleiche FR 1 331 721).

Die insektizide Wirkung dieser Verbindungen ist jedoch insbesondere bei niedrigen Wirkstoffkonzentrationen und Aufwandmengen, nicht immer zufriedenstellend.

Es wurden nun neue S-Azolyl-methyl-di(tri)-thiophosphorsäureester der Formel I

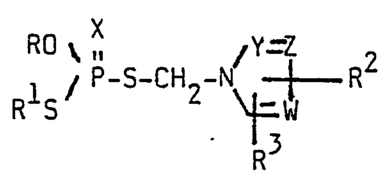

(I)

in welcher

R und $R^1$ gleich oder verschieden sind und für gegebenenfalls substituiertes Alkyl stehen,

$R^2$ und $R^3$ gleich oder verschieden sind und für Wasserstoff, Halogen oder gegebenenfalls substituierte Alkyl-, Alkoxy-, Alkylthio- oder Arylreste stehen,

Y, Z und W gleich oder verschieden sind und für Stickstoff oder einen gegebenenfalls durch $R^2$ oder $R^3$ substituierten Methinrest stehen, und

X für Sauerstoff oder Schwefel steht,

und ihre Säureadditionssalze gefunden.

Die neuen S-Azolyl-methyl-di(tri)-thiophosphorsäureester der Formel (I) und ihre Salze zeichnen sich durch eine hohe Wirksamkeit gegen tierische Schädlinge, insbesondere durch eine hohe insektizide und akarizide Wirksamkeit aus.

Man erhält die neuen Verbindungen der Formel (I) und ihre Salze, wenn man

a) Azolyl-methyl-halogenide der Formel II

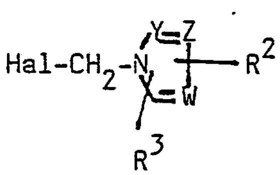

(II)

in welcher

$R^2$, $R^3$, W, Y und Z die oben angegebene Bedeutung haben, und

Hal für Halogen, insbesondere Fluor, Chlor oder Brom steht,

mit Phosphorsäureester-Salzen der Formel III

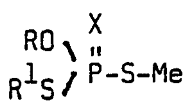

(III)

in welcher

R, $R^1$ und X die oben angegebenen Bedeutungen haben, und

Me für Alkali- oder Erdalkaliionen oder Ammonium steht,

gegebenenfalls in Gegenwart von Verdünnungsmitteln umsetzt, oder

b) Azolyl-methyl-hydroxide der Formel IV

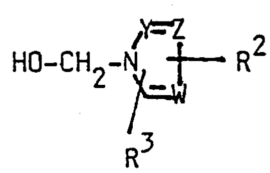

(IV)

in welcher

$R^2$, $R^3$, W, Y und Z die oben angegebene Bedeutung haben,

zunächst mit Sulfonsäurehalogeniden der Formel V

$$R^4-SO_2-Hal \quad (V)$$

in welcher

$R^4$ für Alkyl oder Aryl und

Hal für Halogen, vorzugsweise Chlor oder Brom steht,

gegebenenfalls in Gegenwart von Verdünnungsmitteln und Säureakzeptoren in die Sulfonsäureester der FormelVI.

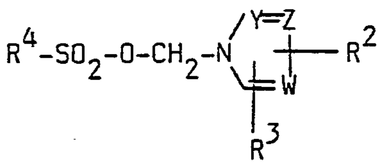

(VI)

in welcher

$R^2$, $R^3$, $R^4$, W, Y und Z die oben angegebene Bedeutung haben,

überführt, und diese dann, gegebenenfalls nach ihrer Isolierung, gegebenenfalls in Gegenwart eines Verdünnungsmittels mit Verbindungen der Formel (III) umsetzt und gewünschtenfalls aus den Verbindungen der Formel (I) in üblicher Weise die Salze herstellt.

Überraschenderweise zeigen die erfindungsgemäßen S-Azolyl-methyl-di(tri)-thiophosphorsäureester der Formel (I) und ihre Salze vorteilhaftere insektizide und akarizide Eigenschaften als entsprechende aus dem Stand der Technik bekannte Verbindungen.

Im gegebenenfalls substituierten Alkyl R, $R^1$, $R^2$, $R^3$ und $R^4$ steht Alkyl für geradkettiges oder verzweigtes Alkyl

mit vorzugsweise 1 bis 6, insbesondere 1 bis 4 Kohlenstoffatomen. Beispielhaft seien gegebenenfalls substituiertes Methyl, Ethyl, n- und iso-Propyl, n-, iso-, sec- und tert-Butyl genannt.

Gegebenenfalls substituiertes Aryl $R^2$, $R^3$ und $R^4$ enthält vorzugsweise 6 bis 10 Kohlenstoffatome. Beispielhaft seien gegebenenfalls substituiertes Phenyl oder Naphthyl genannt.

Im gegebenenfalls substituierten Alkoxy $R^2$ und $R^3$ steht Alkoxy für geradkettiges oder verzweigtes Alkoxy mit vorzugsweise 1 bis 6, insbesondere 1 bis 4 Kohlenstoffatomen. Beispielhaft seien gegebenenfalls substituiertes Methoxy, Ethoxy, n- und iso-Propoxy, n-, iso- und tert-Butoxy, genannt.

Im gegebenenfalls substituierten Alkylthio $R^2$ und $R^3$ steht Alkylthio für geradkettiges oder verzweigtes Alkylthio mit vorzugsweise 1 bis 6, insbesondere 1 bis 4 Kohlenstoffatomen. Beispielhaft seien gegebenenfalls substituiertes Methylthio, Ethylthio, n- und iso-Propylthio, n-, iso- und tert-Butylthio, genannt.

Bei $R^2$ und $R^3$ angegebenes Halogen bedeutet Fluor, Chlor, Brom oder Jod, vorzugsweise Fluor, Chlor oder Brom, insbesondere Chlor oder Brom.

Die bei R, $R^1$, $R^2$, $R^3$ und $R^4$ angegebenen Reste können einen oder mehrere, vorzugsweise 1 bis 3, insbesondere 1 oder 2 gleiche oder verschiedene Substituenten tragen. Als Substituenten seien beispielhaft aufgeführt: Alkyl mit vorzugsweise 1 bis 4, insbesondere 1 oder 2 Kohlenstoffatomen, wie Methyl, Ethyl, n- und iso-Propyl und n-, iso- und tert-Butyl; Alkoxy mit vorzugsweise 1 bis 4, insbesondere 1 oder 2 Kohlenstoffatomen,

wie Methoxy, Ethoxy, n- und iso-Propyloxy und n-, iso- und tert-Butyloxy; Alkylthio mit vorzugsweise 1 bis 4, insbesondere 1 oder 2 Kohlenstoffatomen, wie Methylthio, Ethylthio, n- und iso-Propylthio und n-, iso- und tert-Butylthio; Halogenalkyl mit vorzugsweise 1 bis 4, vorzugsweise 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor oder Brom, insbesondere Fluor oder Chlor stehen, wie Trifluormethyl; Halogen, vorzugsweise Fluor, Chlor, Brom und Iod, insbesondere Fluor, Chlor und Brom; Cyano oder Alkoxycarbonyl mit vorzugsweise 2 bis 4 Kohlenstoffatomen, wie Methoxycarbonyl, Ethoxycarbonyl, n- und iso-Propoxycarbonyl.

Die Erfindung betrifft vorzugsweise Verbindungen der Formel I,

in welcher

R und $R^1$ gleich oder verschieden sind und für gegebenenfalls durch Halogen (vorzugsweise Fluor, Chlor und Brom) substituiertes $C_1$-$C_5$-Alkyl stehen,

$R^2$ und $R^3$ für Wasserstoff, Halogen, wie Fluor, Chlor, Brom oder Iod, vorzugsweise Fluor, Chlor oder Brom, insbesondere Chlor oder Brom oder für einen gegebenenfalls durch Cyano, Halogen wie Fluor, Chlor, Brom oder Iod, vorzugsweise Fluor, Chlor oder Brom, insbesondere Fluor oder Chlor, $C_1$-$C_4$-Alkoxy, $C_1$-$C_4$-Alkylthio oder $C_2$-$C_4$-Alkoxycarbonyl substituierten $C_1$-$C_5$-Alkyl-, $C_1$-$C_5$-Alkoxy oder $C_1$-$C_5$-Alkylthiorest oder für einen gegebenenfalls durch Halogen (vorzugsweise Fluor, Chlor und Brom) substituierten Phenylrest stehen,

X für Sauerstoff oder Schwefel steht,

und in welcher W, Y und Z folgende Bedeutung haben:

a) Y steht für Stickstoff und

Z und W stehen für einen gegebenenfalls durch $R^2$ und $R^3$ substituierten Methinrest,

oder

b) Z steht für Stickstoff und

Y und W stehen für einen gegebenenfalls durch $R^2$ und $R^3$ substituierten Methinrest,

oder

c) Y, W stehen für Stickstoff und

Z steht für einen gegebenenfalls durch $R^2$ substituierten Methinrest mit der Beschränkung, daß $R^3$ nicht für Alkyl steht

und deren Salze.

Besonders bevorzugt sind Verbindungen der Formel I in welcher

R für Methyl oder Ethyl, insbesondere für Ethyl steht,

$R^1$ für Methyl, Ethyl, n-Propyl oder s.-Butyl, insbesondere für Ethyl und n-Propyl steht,

X für Sauerstoff oder Schwefel, insbesondere für Sauerstoff, steht und der Rest der Formel

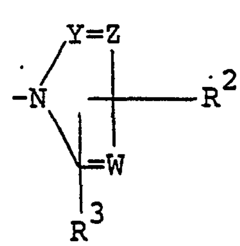

für Imidazol-1-yl, Pyrazol-1-yl, 4-Methoxy-pyrazol-1-yl, 3,5-Dimethyl-pyrazol-1-yl oder 1,2,4-Triazol-1-yl, insbesondere für Imidazol-1-yl, Pyrazol-1-yl oder 1,2,4-Triazol-1-yl steht und deren Salze.

Als Salze der Verbindungen der Formel (I) seien bevorzugt solche mit physiologisch verträglichen Säuren genannt. Beispiele derartiger Säuren sind die Halogenwasserstoffsäuren, wie z.B. die Chlor- und die Bromwasserstoffsäure, insbesondere die Chlorwasserstoffsäure, weiterhin die Phosphorsäure, mono- und bifunktionelle Carbonsäuren und Hydroxycarbonsäuren, wie z.B. Essigsäure, Maleinsäure, Bernsteinsäure, Fumarsäure, Weinsäure, Zironensäure, Salicylsäure, Sorbinsäure, Milchsäure, 1,5-Naphthalindisulfonsäure.

In den Formeln V und VI steht $SO_2-R^4$ für eine Abgangsgruppe, wie sie bei Veresterungsreaktionen vom Typ der Verfahrensvariante (b) üblicherweise eingesetzt werden. $R^4$ steht vorzugsweise für Alkyl mit 1-4 Kohlenstoffatomen, insbesondere für Methyl sowie für Phenyl, welches durch Alkyl, vorzugsweise mit 1-4 Kohlenstoffatomen, insbesondere Methyl substituiert sein kann, wobei der 4-Methylphenylrest (Tolylrest) beispielhaft genannt sei.

Verwendet man bei der erfindungsgemäßen Verfahrensvariante (a) als Ausgangsstoffe beispielweise 1-Chlormethyl-2-methyl-imidazol und O-Ethyl-S-ethyl-dithiophosphorsäurediester-Natriumsalz, so kann der Reaktionsablauf durch folgendes Formelschema skizziert werden:

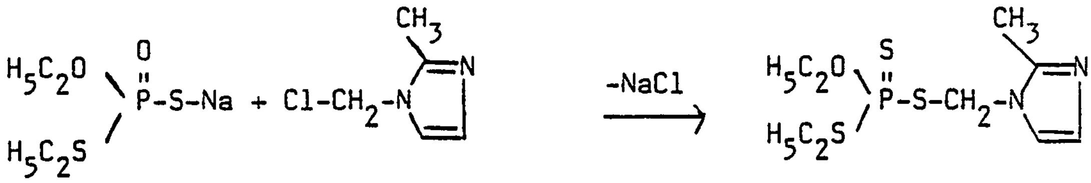


Verwendet man bei der erfindungsgemäßen Verfahrensvariante (b) als Ausgangsstoffe beispielsweise 1-Hydroxymethyl-2-methyl-imidazol, O-Ethyl-S-ethyl-dithiophosphorsäurediester-Natriumsalz, Methansulfonsäurechlorid und Triethylamin, so kann der Reaktionsablauf durch folgendes Formelschema skizziert werden:

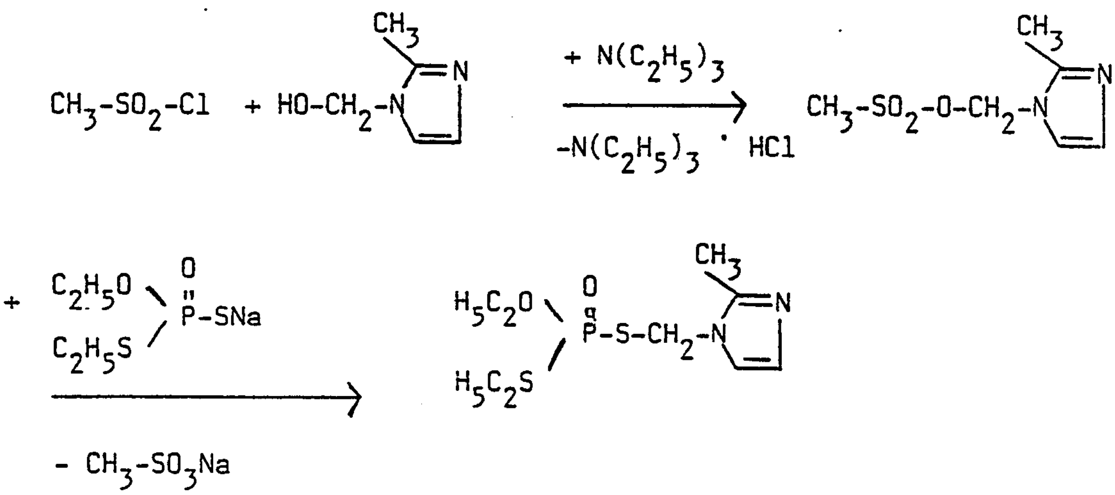


Die als Ausgangsstoffe zu verwendenden Azolyl-methyl-halogenide sind durch die Formel II definiert.

In den Ausgangsstoffen der Formel II haben $R^2$, $R^3$, W, Y und Z die gleichen Bedeutungen, wie sie oben bei Formel I angegeben sind.

Als Beispiele für die Verbindungen der Formel II seien genannt:

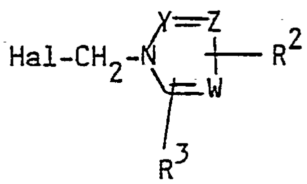

(II)

Hal = Chlor oder Brom

| $R^2$ | $R^3$ | W | Y | Z |
|---|---|---|---|---|
| 3-H | 5-H | -CH | N | $CR^2$ |
| 4-H | 5-H | $-CR^2$ | -CH | -N |
| 3-H | 5-H | -N | -N | $-CR^2$ |
| $3-CH_3$ | $5-CH_3$ | -CH | -N | $-CR^2$ |
| 5-H | $4-CH_3$ | $-CR^3$ | -N | -CH |
| 5-H | $4-OCH_3$ | $-CR^3$ | -N | -CH |
| 5-H | $4-SCH_3$ | $-CR^3$ | -N | -CH |
| 5-H | 4-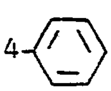 | $-CR^3$ | -N | -CH |
| 5-Cl | 4-Cl | $-CR^3$ | -N | -CH |

| $R^2$ | $R^3$ | W | Y | Z |
|---|---|---|---|---|
| $3-C_2H_5$ | $5-CH_3$ | -CH | -N | $-CR^2$ |
| $3-CH_3$ | $5-C_2H_5$ | -CH | -N | $-CR^2$ |
| 5-Cl | 3-Cl | -CH | -N | $-CR^3$ |
| 5-Cl | 4-Cl | $-CR^3$ | -CH | -N |
| 5-H | $2-CH_3$ | -CH | $-CR^3$ | -N |
| 4-H | $5-CH_3$ | $-CR^2$ | -CH | -N |
| 5-H | 4- 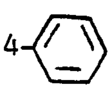 | $-CR^3$ | -CH | -N |
| 5-H | $4-SCH_3$ | $-CR^3$ | -CH | -N |
| 5-H | $4-OCH_3$ | $-CR^3$ | -CH | -N |
| $2-CH_3$ | $4-CH_3$ | $-CR^3$ | $-CR^2$ | -N |
| $3-CH_3$ | 5-H | -N | -N | $-CR^2$ |
| 3-H | 5- 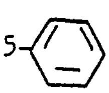 | -N | -N | $-CR^2$ |
| $3-SCH_3$ | 5-H | -N | -N | $-CR^2$ |
| 3-H | $5-SCH_3$ | -N | -N | $-CR^2$ |
| 3-H | $5-OCH_3$ | -N | -N | $-CR^2$ |

| $R^2$ | $R^3$ | W | Y | Z |
|---|---|---|---|---|
| 3-$OCH_3$ | 5-H | -N | -N | -$CR^2$ |
| 3-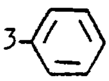 | 5-H | -N | -N | -$CR^2$ |
| 3-Cl | 5-Cl | -N | -N | -$CR^2$ |

Die Verbindungen der Formel II sind bereits bekannt (vergleiche DE-OS 2 835 157 und 2 835 158).

Die als Ausgangsstoffe zu verwendenden Phosphorsäureestersalze sind durch die Formel III definiert.

R, $R^1$ und X haben in den Verbindungen der Formel III die gleichen Bedeutungen, wie sie oben bei Formel I angegeben sind. Me steht für Alkali- oder Erdalkaliionen, insbesondere für Natrium, Kalium oder Calziumionen oder für Ammonium.

Als Beispiele für die Verbindungen der Formel III seien genannt: die Natrium-, Kalium-, Ammonium- oder Calciumsalze der folgenden Phosphorsäureester:
O-Ethyl-S-n-propyl-, O-Ethyl-S-ethyl-, O-Ethyl-S-sek.-butyl-, O-Methyl-S-methyl-, O-Methyl-S-ethyl-, O-n-Propyl-S-ethyl-, O-Ethyl-S-iso-propyl-, O-iso-Propyl-S-ethyl-, O-Methyl-S-iso-propyl-, O-iso-Propyl-S-methyl-, O-Ethyl-S-n-butyl-di(tri)-thiophosphorsäureester.

Die Verbindungen der Formel III sind bereits bekannt. (Vergleiche Houben-Weyl Band 12/2 (1964), 274-292, 607-618).

Die weiter als Ausgangsstoffe zu verwendenden Azolyl-methyl-hydroxide sind durch die Formel IV definiert. In diesen Verbindungen haben $R^2$, $R^3$, W, Y und Z die gleichen Bedeutungen, wie sie oben bei Formel I angegeben sind.

Als Beispiele für die Verbindungen der Formel IV seien genannt:

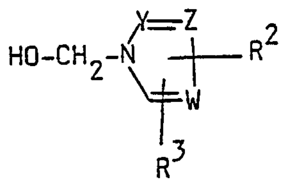

(IV)

| $R^2$ | $R^3$ | W | Y | Z |
|---|---|---|---|---|
| 3-H | 5-H | -CH | -N | $-CR^2$ |
| 4-H | 5-H | $-CR^2$ | -CH | -N |
| 3-H | 5-H | -N | -N | $-CR^2$ |
| $3-CH_3$ | $5-CH_3$ | -CH | -N | $-CR^2$ |
| 5-H | $4-CH_3$ | $-CR^3$ | -N | -CH |
| 5-H | $4-OCH_3$ | $-CR^3$ | -N | -CH |
| 5-H | $4-SCH_3$ | $-CR^3$ | -N | -CH |
| 5-H | 4- 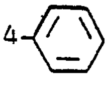 | $-CR^3$ | -N | -CH |
| 5-Cl | 4-Cl | $-CR^3$ | -N | -CH |

| $R^2$ | $R^3$ | W | Y | Z |
|---|---|---|---|---|
| 3-$C_2H_5$ | 5-$CH_3$ | -CH | -N | -$CR^2$ |
| 3-$CH_3$ | 5-$C_2H_5$ | -CH | -N | -$CR^2$ |
| 5-Cl | 3-Cl | -CH | -N | -$CR^3$ |
| 5-Cl | 4-Cl | -$CR^3$ | -CH | -N |
| 5- H | 2--$CH_3$ | -CH | -$CR^3$ | -N |
| 4-H | 5-$CH_3$ | -$CR^2$ | -CH | -N |
| 5-H | 4-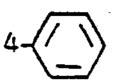 | -$CR^3$ | -CH | -N |
| 5-H | 4-$SCH_3$ | -$CR^3$ | -CH | -N |
| 5-H | 4-$OCH_3$ | -$CR^3$ | -CH | -N |
| 2-$CH_3$ | 4-$CH_3$ | -$CR^3$ | -$CR^2$ | -N |
| 3-$CH_3$ | 5-H | -N | -N | -$CR^2$ |
| 3-H | 5-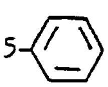 | -N | -N | -$CR^2$ |
| 3-$SCH_3$ | 5-H | -N | -N | -$CR^2$ |
| 3-H | 5-$SCH_3$ | -N | -N | -$CR^2$ |
| 3-H | 5-$OCH_3$ | -N | -N | -$CR^2$ |
| 3-$OCH_3$ | 5-H | -N | -N | -$CR^2$ |

| $R^2$ | $R^3$ | W | Y | Z |
|---|---|---|---|---|
| 3-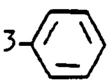 | 5-H | -N | -N | $-CR^2$ |
| 3-Cl | 5-Cl | -N | -N | $-CR^2$ |

Azolyl-methyl-hydroxide der Formel IV sind bekannt (vergleiche DE-OS 2 835 157 und 2 835 158).

Die Verfahrensvarianten (a) und (b) zur Herstellung von Verbindungen der Formel (I) werden vorzugsweise unter Verwendung von Verdünnungsmitteln durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid, und N-Methyl-pyrrolidon, sowie Dimethylsulfoxid, Tetramethylensulfon und Hexamethylphosphorsäuretriamid.

Der erste Reaktionsschritt der erfindungsgemäßen Verfahrensvariante (b) wird vorzugsweise in Gegenwart von Säureakzeptoren durchgeführt. Als Säureakzeptoren können die üblichen Säurebindemittel Verwendung finden. Besonders bewährt haben sich Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Diazabicyclooctan, Diazabicyclononen und Diazabicycloundecen.

Die erfindungsgemäße Verfahrensvariante (b) wird unter Verwendung von Sulfonsäurehalogeniden der Formel (V) durchgeführt. Vorzugsweise werden Benzol-, p-Toluol- und Methansulfonsäure-chlorid bzw. -bromid eingesetzt.

Die erfindungsgemäßen Verfahrensvarianten (a) und (b) werden im allgemeinen bei Normaldruck durchgeführt.

Die Reaktionstemperatur kann bei den Verfahrensvarianten (a) und (b) innerhalb eines größeren Bereichs variiert werden. Im allgemeinen arbeitet man zwischen 0 und 100°C, vorzugsweise bei 0 bis 60°C.

Zur Durchführung der erfindungsgemäßen Verfahrensvarianten (a) und (b) werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel durchgeführt und das Reaktionsgemisch wird mehrere Stunden bei der erforderlichen Temperatur gerührt. Danach wird nach allgemein üblichen Methoden das Reaktionsgemisch aufgearbeitet und das gewünschte Produkt isoliert. Hierzu wird z.B. das Lösungsmittel unter vermindertem Druck abdestilliert, wobei gebildete Salze zuvor, z.B. durch Ausschütteln mit Wasser, entfernt werden. Die Produkte können durch ihre Schmelzpunkte oder Brechungsindices charakterisiert werden.

Bei der Verfahrensvariante (b) kann der Sulfonsäureester der Formel VI nach üblichen Methoden isoliert und dann mit den Verbindungen der Formel III umgesetzt werden. Vorteilhaft ist es jedoch, ohne die Isolierung der Verbindung VI zu arbeiten .

Die Verbindungen der Formel I können nach den allgemein üblichen Methoden in die Säureadditionssalze übergeführt werden, z.B. durch Auflösen in einem Lösungsmittel, z.B. Diethyläther und Zugabe von HCl-Gas, worauf das Hydrochlorid ausfällt und abgesaugt werden kann.

Die Wirkstoffe (Verbindungen der Formel I und/oder ihre Salze) eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- u Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem Gebiet der Tierhaltung und Viehzucht, wobei durch die Bekämpfung der Schädlinge bessere Ergebnisse, z.B. höhere Milchleistungen, höheres Gewicht, längere Lebensdauer usw. erreicht werden können.

Die Anwendung der erfindungsgemäßen Wirkstoffe geschieht auf diesen Gebieten in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die Wirksamkeit der erfindungsgemäßen Verbindungen sei anhand der folgenden Beispiele erläutert, wobei mit den beiden in der Einleitung genannten Verbindungen der FR-Patentschrift 1 331 721 ("Vergleichsverbindungen") verglichen wurde:

Beispiel A

Phaedon-Larven-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0 % bedeutet, daß keine Käfer-Larven abgetötet wurden.

Bei diesem Test zeigten beispielsweise bei einer Wirkstoffkonzentration von 0,01% nach 3 Tagen die Verbindungen der Beispiele 1, 2 und 3 eine 100%ige Abtötung, während die oben erwähnten Verbindungen des Standes der Technik keine Abtötung (0%) ergaben.

Beispiel B

Tetranychus-Test (resistent)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigten z.B. bei einer Wirkstoffkonzentration von 0,1% nach 2 Tagen die Verbindungen der Beispiele 1, 2 und 3 eine Abtötung von 100 %.

Beispiel C

Test mit Lucilia cuprina res.-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 2o Lucilia cuprina res.-Larven werden in ein Teströhrchen gebracht, welches ca. 1 $cm^2$ Pferdefleisch und o,5 ml der Wirkstoffzubereitung enthält. Nach 24 Stunden wird der Abtötungsgrad bestimmt.

Bei diesem Test zeigte z.B. die Verbindung des Beispiels 1 bei einer Wirkstoffkonzentration von 10 ppm eine 100%ige Abtötung.

Die erfindungsgemäßen Verfahrensvarianten (a) und (b) sollen anhand der folgenden Beispiele erläutert werden:

Beispiel 1

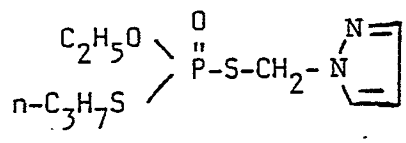

Verfahrensvariante a:

Eine Mischung aus 11,6 g (o,1 Mol) 1-Chlormethylpyrazol, 15o ml Acetonitril und 23,8 g (o,1 Mol) O-Ethyl-S-n-propyl-dithiophosphorsäurediester-Kaliumsalz wird 12 Stunden bei 2o°C gerührt. Nach Zugabe von 4oo ml Toluol wird zweimal mit je 3oo ml Wasser ausgeschüttelt, dann trocknet man die organische Phase über Natriumsulfat, dampft das Lösungsmittel im Vakuum ab und destilliert den Rückstand bei 6o°C im Hochvakuum an. Man erhält so 23,8 g (85% der Theorie) O-Ethyl-S-n-propyl-S-pyrazol-1-yl-methyl-dithiophosphorsäureester in Form eines gelben Öles mit dem Brechungsindex $n_D^{21}$: 1,5448.

Verfahrensvariante b:

Zu einer Mischung aus 14,85 g (o,15 Mol) 1-Hydroxymethylpyrazol, 16,5 g (o,165 Mol) Triethylamin und 4oo ml Acetonitril tropft man unter leichter Kühlung bei 1o-15°C 17,2 g (o,15 Mol) Methansulfonsäurechlorid und rührt dann eine halbe Stunde bei Raumtemperatur nach. Anschließend versetzt man das Reaktionsgemisch mit 35,7 g (o,15 Mol) O-Ethyl-S-n-propyl-dithiophosphorsäurediester-Kaliumsalz und rührt 16 Stunden bei 2o°C nach. Nach Zugabe von 5oo ml Toluol schüttelt man 3 mal mit je 3oo ml Wasser, trocknet die organische Phase über Natriumsulfat und destilliert das Lösungsmittel im Vakuum ab. Der Rückstand wird bei 6o°C im Hochvakuum andestilliert. Zurück bleiben 24 g (58% der Theorie) der oben genannten Verbindung.

In analoger Weise können die folgenden Verbindungen der Formel

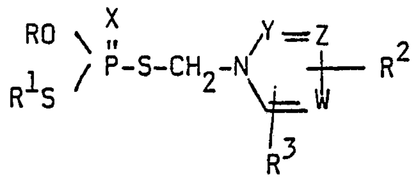

(I)

erhalten werden:

| Bei-spiel Nr. | R | $R^1$ | $R^2$ | $R^3$ | X | W | Y | Z | Ausbeute (% der Theorie) | Physikal. Daten Schmelzpunkt (°C); Brechungsindex |
|---|---|---|---|---|---|---|---|---|---|---|
| 2 | $C_2H_5$ | n-$C_3H_7$ | 3-H | 5-H | S | CH | N | $CR^2$ | 85 | $n_D^{20}$: 1,5762 |
| 3 | $C_2H_5$ | n-$C_3H_7$ | 3-H | 5-H | O | N | N | $CR^2$ | 76 | $n_D^{20}$: 1,5411 |
| 4 | $C_2H_5$ | n-$C_3H_7$ | 4-H | 5-H | O | $CR^2$ | CH | N | 17 | $n_D^{24}$: 1,5493 |
| 5 | $C_2H_5$ | $C_2H_5$ | 3-H | 5-H | S | CH | N | $CR^2$ | 64 | $n_D^{20}$: 1,5764 |
| 6 | $C_2H_5$ | n-$C_3H_7$ | 3-H | 5-H | S | N | N | $CR^2$ | 75 | $n_D^{24}$: 1,5742 |
| 7 | $C_2H_5$ | n-$C_3H_7$ | 4-H | 5-H | S | $CR^2$ | CH | N | 38 | $n_D^{24}$: 1,5912 |
| 8 | $C_2H_5$ | sek-$C_4H_9$ | 3-H | 5-H | O | CH | N | $CR^2$ | | |
| 9 | $C_2H_5$ | sek-$C_4H_9$ | 4-H | 5-H | O | $CR^2$ | CH | N | | |
| 1o | $C_2H_5$ | sek-$C_4H_9$ | 3-H | 5-H | O | N | N | $CR^2$ | | |
| 11 | $CH_3$ | $CH_3$ | 3-H | 5-H | O | CH | N | $CR^2$ | | |
| 12 | $C_2H_5$ | n-$C_3H_7$ | 3-$CH_3$ | 5-$CH_3$ | O | CH | N | $CR^2$ | | |

| Beispiel Nr. | R | $R^1$ | $R^2$ | $R^3$ | X | W | Y | Z | Ausbeute (% der Theorie) | Physikal. Daten Schmelzpunkt ($^oC$) Brechungsindex; |
|---|---|---|---|---|---|---|---|---|---|---|
| 13 | $C_2H_5$ | $n-C_3H_7$ | $4-OCH_3$ | 5-H | O | $CR^2$ | N | CH | 59 | $n_D^{24}$: 1,5373 |
| 14 | $C_2H_5$ | $n-C_3H_7$ | $4-OCH_3$ | 5-H | S | $CR^2$ | N | CH | | |
| 15 | $C_2H_5$ | $n-C_3H_7$ | $3-CH_3$ | $5-CH_3$ | S | CH | N | $CR^2$ | | |

Patentansprüche

1. Phosphorsäureester der Formel I

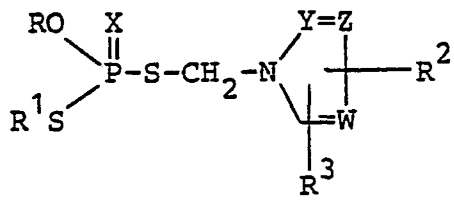

(I)

in welcher

R und $R^1$ gleich oder verschieden sind und für gegebenenfalls substituiertes Alkyl stehen,

$R^2$ und $R^3$ gleich oder verschieden sind und für Wasserstoff, Halogen oder gegebenenfalls substituierte Alkyl-, Alkoxy-, Alkylthio- oder Arylreste stehen,

Y, Z und W gleich oder verschieden sind und für Stickstoff oder einen gegebenenfalls durch $R^2$ oder $R^3$ substituierten Methinrest stehen, und

X für Sauerstoff oder Schwefel steht,

und ihre Säureadditionssalze.

2. Phosphorsäureester gemäß Anspruch 1, wobei in Formel I

R und $R^1$ gleich oder verschieden sind und für gegebenenfalls durch Halogen substituiertes $C_1$-$C_5$-Alkyl stehen,

$R^2$ und $R^3$ für Wasserstoff, Halogen, wie Fluor, Chlor,Brom oder Iod, vorzugsweise Fluor, Chlor oder Brom, insbesondere Chlor oder Brom oder für einen gegebenenfalls durch Cyano, Halogen wie Fluor, Chlor, Brom oder Iod, vorzugsweise Fluor, Chlor oder Brom, insbesondere Fluor oder Chlor, $C_1$-$C_4$-Alkoxy, $C_1$-$C_4$-Alkylthio oder $C_2$-$C_4$-Alkoxycarbonyl substituierten $C_1$-$C_5$-Alkyl-, $C_1$-$C_5$-Alkoxy- oder $C_1$-$C_5$-Alkylthiorest oder für einen gegebenenfalls durch Halogen substituierten Phenylrest stehen,

X für Sauerstoff oder Schwefel steht,

und wobei W, Y und Z folgende Bedeutungen haben:

a) Y steht für Stickstoff und

Z und W stehen für einen gegebenenfalls durch $R^2$ und $R^3$ substituierten Methinrest,

oder

b) Z steht für Stickstoff und

Y und W stehen für einen gegebenenfalls durch $R^2$ und $R^3$ substituierten Methinrest,

oder

c) Y, W stehen für Stickstoff und

Z steht für einen gegebenenfalls durch $R^2$ substituierten Methinrest mit der Beschränkung, daß $R^3$ nicht für Alkyl steht

und ihre Säureadditionssalze.

3. Phosphorsäureester gemäß den Ansprüchen 1 und 2, wobei in Formel I

R für Methyl oder Ethyl steht,

$R^1$ für Methyl, Ethyl, n-Propyl oder s.-Butyl steht,

X für Sauerstoff oder Schwefel steht, und der Rest der Formel

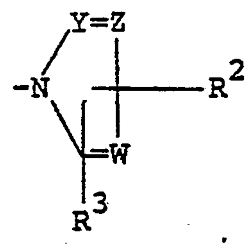

für Imidazol-1-yl, Pyrazol-1-yl, 4-Methoxy-pyrazol-1-yl, 3,5-Dimethyl-pyrazol-1-yl oder 1,2,4-Triazol-1-yl steht,

und ihre Säureadditionssalze.

4. Phosphorsäureester der Formel

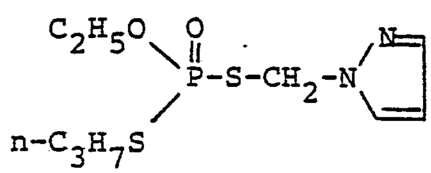

und seine Säureadditionssalze.

5. Phosphorsäureester der Formel

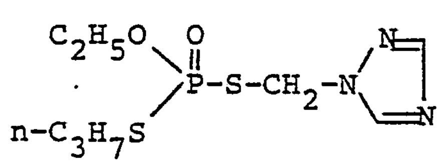

und seine Säureadditionssalze.

6. Verfahren zur Herstellung von Phosphorsäureestern der Formel I

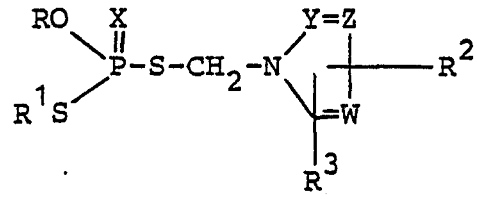

(I)

in welcher

R und $R^1$ gleich oder verschieden sind und für gegebenenfalls substituiertes Alkyl stehen,

$R^2$ und $R^3$ gleich oder verschieden sind und für Wasserstoff, Halogen oder gegebenenfalls substituierte Alkyl-, Alkoxy-, Alkylthio- oder Arylreste stehen,

Y, Z und W gleich oder verschieden sind und für Stickstoff oder einen gegebenenfalls durch $R^2$ oder $R^3$ substituierten Methinrest stehen, und

X für Sauerstoff oder Schwefel steht,

und ihre Säureadditionssalze, dadurch gekennzeichnet, daß man

a) Azolyl-methyl-halogenide der Formel II

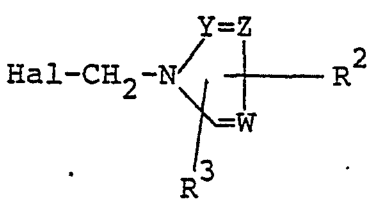

(II)

in welcher

$R^2$, $R^3$, W, Y und Z die oben angegebene Bedeutung haben, und

Hal für Halogen steht,

mit Phosphorsäureester-Salzen der Formel III

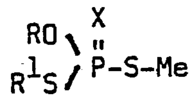

(III)

in welcher

R, $R^1$ und X die oben angegebenen Bedeutungen haben, und

Me für Alkali- oder Erdalkaliionen oder Ammonium steht,

gegebenenfalls in Gegenwart von Verdünnungsmitteln umsetzt, oder

b) Azolyl-methyl-hydroxide der Formel IV

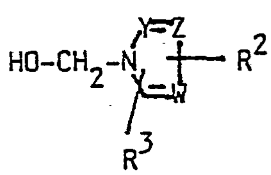

(IV)

in welcher

$R^2$, $R^3$, W, Y und Z die oben angegebene Bedeutung haben,

zunächst mit Sulfonsäurehalogeniden der Formel V

$$R^4-SO_2-Hal \qquad (V)$$

in welcher

$R^4$ für Alkyl oder Aryl und

Hal für Halogen steht,

gegebenenfalls in Gegenwart von Verdünnungsmitteln und Säureakzeptoren in die Sulfonsäureester der FormelVI.

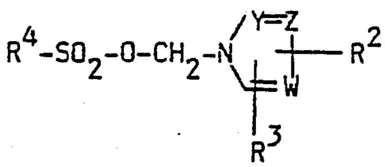

(VI)

in welcher

$R^2$, $R^3$, $R^4$, W, Y und Z die oben angegebene Bedeutung haben,

überführt, und diese dann, gegebenenfalls nach ihrer Isolierung, gegebenenfalls in Gegenwart eines Verdünnungsmittels mit Verbindungen der Formel (III) umsetzt und gewünschtenfalls aus den Verbindungen der Formel I das Salz herstellt.

7. Schädlingsbekämpfungsmittel, gekennzeichnet durch einen Gehalt an mindestens einem Phosphorsäureester der Formel I und/oder seines Säureadditionssalzes.

8. Verwendung von Phosphorsäureestern der Formel I und/oder ihrer Säureadditionssalze zur Bekämpfung von Schädlingen, insbesondere Insekten und Spinnentieren.

9. Verfahren zur Bekämpfung von Schädlingen, dadurch gekennzeichnet, daß man Phosphorsäureester der Formel I und/oder ihre Säureadditionssalze auf die Schädlinge, vorzugsweise Insekten oder Spinnentiere oder ihren Lebensraum einwirken läßt.

10. Verfahren zur Herstellung von Schädlingsbekämpfungsmitteln, dadurch gekennzeichnet, daß man Phosphorsäureester der Formel I und/oder ihre Säureadditionssalze mit Streckmitteln und/oder oberflächenaktiven Mitteln vermischt.

Europäisches Patentamt

# EUROPÄISCHER RECHERCHENBERICHT

0069881

Nummer der Anmeldung

EP 82 10 5413

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 133 571 (CIBA-GEIGY AG.) * das ganze Dokument * --- | 1-10 | C 07 F 9/65<br>A 01 N 57/16 |
| D,Y | FR-A-1 331 721 (J.R. GEIGY S.A.) * das ganze Dokument * ----- | 1-10 | |
| | | | **RECHERCHIERTE SACHGEBIETE (Int. Cl. 3)** |
| | | | C 07 F 9/00<br>A 01 N 57/00 |

Der vorliegende Recherchenbericht wurde für alle Patentansprüche erstellt.

| Recherchenort | Abschlußdatum der Recherche | Prüfer |
|---|---|---|
| DEN HAAG | 08-10-1982 | BESLIER L.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82